# EUROPÄISCHE PATENTANMELDUNG

(11) **EP 1 941 817 A1**
(43) Veröffentlichungstag der Anmeldung: **09.07.2008**
(21) Anmeldenummer: 07000155.7
(22) Anmeldetag: 05.01.2007
(51) Int. Cl.: A47G 9/02, A61F 7/02

(54) **Wärmedecke**

(71) Anmelder: Moeck, Lidia, 22607 Hamburg (DE)
(72) Erfinder: Moeck, Lidia, 22607 Hamburg (DE)
(74) Vertreter: Richter, Werdermann, Gerbaulet & Hofmann

(57) **Zusammenfassung**

Die vorliegende Erfindung betrifft eine Wärmedecke (100) insbesondere eine Wärmeunterlage, aufweisend
- mindestens eine dem mittels der Wärmedecke (100) zu wärmenden Körper zugewandte, für die Warmluft durchlässig ausgebildete Innenschicht (14); und
- mindestens eine Zwischenlage oder Zwischenschicht (16) zum Durchlassen, zum Durchleiten, zum Durchströmen und/oder zum Transportieren der Warmluft.

Um bei Wärmedecken eine Außenschicht, die sich aus Polyester und Polyurethan zusammensetzt, zu ersetzen, schlägt die Erfindung vor, dass eine Beschichtung auf die Zwischenschicht (16) aufgetragen ist.

## Beschreibung

### Technisches Gebiet

Die vorliegende Erfindung betrifft eine Wärmedecke, insbesondere eine Wärmeunterlage, aufweisend
- mindestens eine dem mittels der Wärmedecke zu wärmenden Körper zugewandte, für die Warmluft durchlässig ausgebildete Innenschicht; und
- mindestens eine Zwischenlage oder Zwischenschicht zum Durchlassen, zum Durchleiten, zum Durchströmen und/oder zum Transportieren der Warmluft.

### Stand der Technik

Wärmedecken der eingangs genannten Art dienen grundsätzlich der Temperierung eines auf der Wärmedecke gelagerten und/oder von der Wärmedecke bedeckten Körpers, indem Warmluft zugeführt wird. Derartige Wärmedecken finden insbesondere, aber nicht ausschließlich im medizinischen Bereich Anwendung, um Wärmeverluste von zu behandelnden Personen zu verhindern, deren selbständige Wärmeregulation gestört oder vorübergehend außer Kraft gesetzt ist.

Die Wärmedecke wird hierbei unter und/oder über den Körper des Patienten oder des Patienten gelegt und die durch mindestens einen Zuführschlauch zugeleitete Warmluft tritt flächig verteilt an der dem Körper zugewandten Innenseite der Wärmedecke aus, um den Körper zu erwärmen.

Die Verwendung solcher Wärmedecken trägt der Tatsache Rechnung, dass gerade im Bezug auf Operationen, d. h. im medizinischen und speziell im chirurgischen Bereich ein dringender Bedarf an kontinuierlicher und "sanfter" Wärmezufuhr zum menschlichen Körper besteht, denn durch die bei einer Operation im Regelfall erforderliche Teil- oder Voll-Narkotisierung werden grundlegende Körperfunktionen partiell oder vollständig für die Dauer der Narkotisierung außer Kraft gesetzt, so dass die Gefahr einer gefährlichen Auskühlung bzw. Unterkühlung des Körpers sehr hoch ist.

Zwischen der dem Körper zugewandten, für die Warmluft durchlässig ausgebildeten Innenschicht und einer vom Körper abgewandten, für die Warmluft im Wesentlichen undurchlässig ausgebildeten Außenschicht ist mindestens eine Zwischenschicht angeordnet. Dies bedeutet, dass der innenliegende Raum oder "Innenraum" der Wärmedecke, insbesondere der Wärmeunterlage, mit einem Material gefüllt ist, das nicht nur einen Warmluftdurchlass sicherstellt, sondern auch ein druckentlastendes Lagern von narkotisierten und wachen Patientinnen und/oder Patienten gewährleistet.

Die Wärmeunterlage wird so mit ihrer dem Körper zugewandten Innenschicht auf und unter den Körper gelegt und isoliert diesen nicht nur, sondern wärmt diesen konvektiv, d.h. mit ausströmender Warmluft. Durch eben diese konvektive Wärmezufuhr wird eine Auskühlung bzw. Unterkühlung auch eines zu operierenden Körpers in zuverlässiger Weise verhindert. Eine leichte Kühlung ist jedoch möglich.

Die aus dem Stand der Technik bekannten Wärmedecken zeichnen sich demnach vornehmlich durch folgende Struktur aus:
- als Innenschicht mindestens eine Mikrofaserschicht, die luftdurchlässig, d.h. für die Warmluft permeabel ausgebildet ist;
- als Zwischenschicht mindestens ein Abstands-/ Lagerungsgewebe oder -gewirke, insbesondere mindestens ein Gewebe aus Chemiefaser oder ein Polyestergewebe oder -gewirke, zum Durchlassen, zum Durchleiten, zum Durchströmen und/oder zum Transportieren der Warmluft;
- als Außenschicht mindestens eine Textilschicht, die luftundurchlässig, d.h. für die Warmluft impermeabel ausgebildet ist. Bei der Außenschicht handelt es sich um eine separate Schicht, die sich vornehmlich aus Polyester und Polyurethanbeschichtung zusammensetzt.

Es hat sich gezeigt, dass die dreigliedrige Ausgestaltung der Wärmedecke, d.h. die Herstellung einer Innenschicht, einer Zwischenschicht sowie einer Außenschicht als unter Umständen zusammengenähte voneinander getrennte Schichten, einen aufwendigen Herstellungsprozess erfordert und zudem materialintensiv ist. Auf der anderen Seite bleibt festzuhalten, dass die Zwischenschicht aufgrund der Gewährleistung eines druckentlastenden Lagerns der Patientinnen bzw. Patienten eine Funktion erfüllt, auf die nicht verzichtet werden kann. Dadurch, dass die Innenschicht aus einem luftdurchlässigen Material besteht, ist auch die Innenschicht von essenzieller Bedeutung. Zudem wird für die Funktionalität der Wärmedecke auch eine luft- und feuchtigkeitsundurchlässige Außenschicht benötigt, die in erster Linie durch das in die Außenschicht integrierte Polyurethan gewährleistet ist.

### Darstellung der Erfindung, Aufgabe, Lösung, Vorteile

Ausgehend von den dargelegten Nachteilen und Unstimmigkeiten sowie unter Würdigung des aufgezeigten Standes der Technik bei Wärmedecken liegt der vorliegenden Erfindung die Aufgabe zugrunde, eine Wärmedecke der eingangs genannten Art so auszubilden, dass unter Wahrung der Funktionalität der Wärmedecke auf eine separate Schicht verzichtet werden kann.

Diese Aufgabe wird durch eine Wärmedecke mit den in Anspruch 1 angegebenen Merkmalen gelöst. Vorteilhafte Ausgestaltungen und zweckmäßige Weiterbildungen der vorliegenden Erfindung sind in den Unteransprüchen gekennzeichnet.

Die grundlegende Idee der Erfindung ist es, auf die Zwischenschicht einer Wärmedecke eine Beschichtung direkt aufzutragen. Diese Beschichtung kann insbesondere aus Polyurethan sein. Auf diese Weise entfällt bei der Wärmedecke der eingangs genannten Art eine weitere separate Schicht, die sich aus Polyester und Polyurethan zusammensetzt. Gleichzeitig ist jedoch die Funktion einer separaten mit der Zwischenschicht zusammengenähte Außenschicht gegeben, indem beispielsweise mit Polyurethan ein Material gegeben ist, welches luft- und wasserundurchlässig ist. Durch das Auftragen des Polyurethans auf die Zwischenschicht wird somit eine Beschichtung geschaffen, die die separate Außenschicht einer Wärmedecke ersetzt. Die Aufbringung der Polyurethanschicht kann dabei in üblicher Weise im Streich- oder Umkehrverfahren erfolgen. Auf diese Weise geht das Polyurethan eine direkte Verbindung mit der Zwischenschicht ein, die sich beispielsweise aus Gewirkware zusammensetzen kann.

Gemäß einer bevorzugten Weiterbildung der vorliegenden Erfindung handelt es sich bei der Zwischenschicht um mindestens ein Kunststoffgewebe oder - gewirke, dass einerseits elastisch genug ist, um im Hinblick auf die Verformungseigenschaften bei Druckspannung ein angenehmes sowie druckentlastendes Liegen zu garantieren, und das andererseits so starr ist, dass es sich nur bedingt zusammendrücken lässt, sodass ein Durchströmen mit Warmluft gewährleistet ist. Der Fachmann auf dem Gebiet der Wärmedecken, insbesondere der Wärmeunterlagen, beispielsweise ein Fachhochschulingenieur auf dem Gebiet der Textiltechnik mit speziellen Kenntnissen im Bereich der Wärmelehre wird des Weiteren im Bezug auf die Lehre der vorliegenden Erfindung zu schätzen wissen, dass die beiden anderen Lagen oder Schichten (nämlich die Innenlage oder Innenschicht sowie die Außenlage oder Außenschicht, die als Beschichtung auf die Zwischenschicht aufgetragen ist) durch das Anordnen der Zwischenlage oder Zwischenschicht nicht in einem Maße zusammengedrückt werden können, dass ein Durchlassen, ein Durchleiten, ein Durchströmen und/oder ein Transportieren der Warmluft nicht mehr ermöglichen würde (die seitlichen Körperpartien werden ebenfalls durch die aus der Innenlage oder Innenschicht ausströmende Warmluft gewärmt).

Eine praktikable Variante der Erfindung sieht vor, dass ein Polyurethanschaum auf die Zwischenschicht aufgetragen ist. Ein weiterer Gedanke der Erfindung ist es, die Polyurethanbeschichtung mit dem Einsatz von Polyurethanschaum zu kombinieren.

Dabei kann zunächst das Polyurethan in einer gewissen Schichtdicke auf die Zwischenschicht aufgetragen werden, wobei anschließend der Polyurethanschaum zur Ausbildung eines Kerns aufgebracht wird, um dann erneut eine dünne Schicht Polyurethan aufzutragen. Es entsteht so gewissermaßen ein Sandwichaufbau. Mit dem Auftrag einer ersten Polyurethanschicht wird die Zwischenschicht komplett beschichtet, um hierdurch zunächst eine luftundurchlässige Schicht zu schaffen. Mit dem Polyurethanschaum ist es in Kurzzeit möglich, größere Schichten aufzubauen. Dies kann mit einer Polyurethanbeschichtung, beispielsweise im Streich- oder Umkehrverfahren, nicht bewerkstelligt werden. Der Auftrag einer weiteren Polyurethanschicht dient dann der Ausbildung einer glatten Oberfläche sowie der Sicherung der Dichtheit des Sandwichaufbaus. Denkbar ist aber auch, dass der Polyurethanschaum direkt auf die Zwischenschicht aufgetragen wird. Dadurch, dass der Polyurethanschaum direkt auf die Zwischenschicht aufgetragen wird, ist ein schneller Aufbau einer Schicht gewährleistet.

Bei der Herstellung des Sandwichaufbaus kann derart vorgegangen werden, dass zunächst die Polyurethanschicht, beispielsweise im Streich- oder Umkehrverfahren, fertig gestellt wird. Sodann wird in einem nächsten Schritt der Polyurethanhartschaum, beispielsweise durch flüssige Roh-Komponenten, hergestellt. Schließlich wird das Polyurethanhartschaumgemisch auf die Polyurethanschicht aufgetragen und ausgehärtet. Zur Ausbildung des Sandwichverbundes kann dann noch in einem weiteren Schritt eine weitere Polyurethanschicht auf dem Polyurethanschaum aufgetragen werden. Da die Wärmedecke auf ihrer vom Körper abgewandten Schicht wärmereflektierend ausgebildet sein sollte, ist es von Vorteil, dass die Polyurethanschicht bzw. der Sandwichaufbau sich durch eine weiße Färbung auszeichnet.

Im Rahmen der vorliegenden Erfindung weist die Wärmedecke
- mindestens einen Kopfbereich bzw. mindestens ein Kopfteil und/oder
- mindestens einen Armbereich bzw. mindestens ein Armteil und/oder
- mindestens einen Brustbereich bzw. mindestens ein Brustteil und/oder
- mindestens einen Unterkörperbereich bzw. mindestens ein Unterkörperteil und/oder
- mindestens ein Beinbereich bzw. mindestens ein Beinteil und/oder
- mindestens ein Seitenteil und/oder
- mindestens ein Taschenteil auf.

Diese Teile ermöglichen ein optimales Anpassen der Wärmedecke an die körperlichen Gegebenheiten der Patientin bzw. des Patienten. So können z.B. einzelne Arme und/oder einzelne Beine auch in taschenartig ausgebildete Teile eingesteckt werden. Auch ist es möglich, dass ganze Körperpartien abgedeckt werden.

Eine vorteilhafte Ausgestaltung der Erfindung sieht vor, dass die Wärmedecke mindestens einen Kopfbereich, der mit dem Kopf und/oder mit dem Hals einer von der Wärmedecke bedeckten Person in Kontakt gebracht werden kann, umfasst, und den Kopf und/oder den Hals durch die zugeführte Warmluft zu erwärmen.

Die Wärmedecke gem. der vorliegenden Erfindung lässt somit den Kopfbereich und/oder den Halsbereich einer Patientin oder eines Patienten nicht offen, sondern bezieht auch diese Bereiche mit in die Isolierung und Wärmezufuhr ein. Auf diese Weise kann eine erhebliche Verbesserung der Temperierungseigenschaften der vorliegenden Wärmedecke erzielt werden.

Eine praktikable Variante der Erfindung sieht vor, dass die Wärmedecke mindestens einen Armbereich umfasst, der den Arm oder einen Teil des Armes einer von der Wärmedecke bedeckten Person umschließend angeordnet werden kann.

Durch den Armbereich bzw. durch die vorzugsweise zwei Armbereiche können auch die Arme einer Patientin bzw. eines Patienten ganz oder teilweise, etwa lediglich im Oberarmbereich und/oder lediglich im Unterarmbereich, in die Wärmeregulierung einbezogen werden. Auch diese Maßnahme führt zu einer deutlichen Verbesserung des Wärmeschutzes, denn auch über den (Ober- und/oder Unter-) Armbereich kann ein beachtlicher Wärmeverlust eintreten, weil die Arme - wie generell alle Extremitäten - gegenüber dem Rumpf der Gefahr eines höheren Grads an Auskühlung bzw. an Unterkühlung unterliegen.

Auch kann die Wärmedecke mindestens einen Oberkörperbereich aufweisen, der zumindest partiell mit der Brust und/oder zumindest partiell mit dem Rücken einer auf der Wärmedecke zu lagernden und/oder von der Wärmedecke zu bedeckenden Person zum Erwärmen mittels austretender Warmluft in Kontakt bringbar ist, insbesondere zumindest die Brust und/oder zumindest partiell den Rücken umschließend anordbar ist.

Schließlich sieht die Erfindung vor, dass die Wärmedecke mindestens einen Unterkörperbereich aufweist, der zumindest partiell mit dem Unterkörper und/oder zumindest partiell mit mindestens einem Bein, insbesondere zumindest partiell mit einem Fuß, einer auf der Wärmedecke zu lagernden und/oder von der Wärmedecke zu bedeckenden Person zum Erwärmen mittels austretender Warmluft in Kontakt bringbar ist, insbesondere partiell den Unterkörper und/oder zumindest partiell das Bein bzw. zumindest partiell den Fuß umschließend anordbar ist.

### Kurze Beschreibung der Zeichnungen

Im Folgenden wird die Erfindung anhand der Figuren näher erläutert. Es zeigen in schematischen Darstellungen:
- Fig. 1: eine Aufsicht auf eine Wärmedecke gemäß der Erfindung,
- Fig. 2 a) und b): eine Schnittaussicht der Wärmedecke aus Fig. 1 entlang der Schnittlinie II - II aus Fig. 1

### Bester Weg zur Ausführung der Erfindung

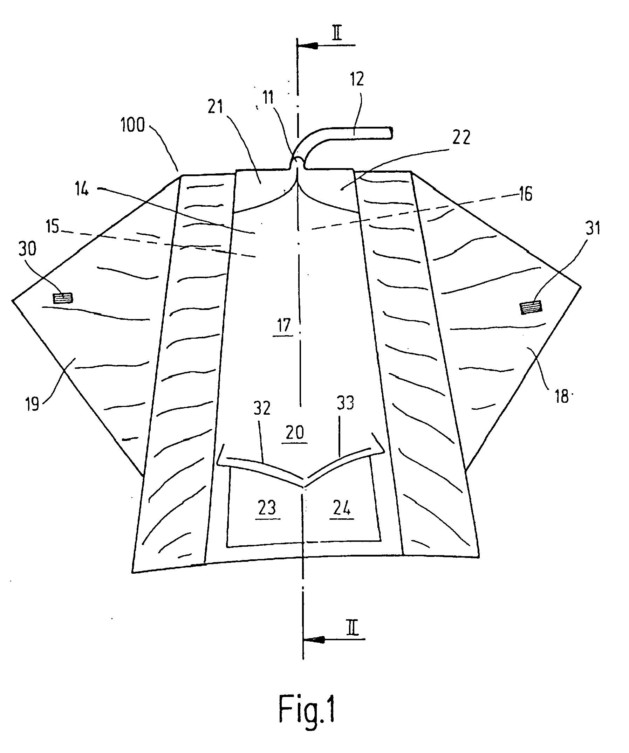

In Figur 1 ist ein Ausführungsbeispiel einer für den Körper eines Lebewesens, insbesondere eines Menschen, bestimmten erfindungsgemäßen Wärmedecke 100 dargestellt, die jeweils als Wärmeunterlage zum Beispiel bei Operationen am Körper zum Einsatz gelangen kann.

Die Wärmedecke 100 weist einen Anschluss 11 für einen Luftschlauch 12 auf, über den Warmluft aus einer Warmluftquelle, wie etwa aus einem strombetriebenen Heizgebläse oder Lufterwärmungsgerät, zugeführt werden kann.

Die erfindungsgemäße Wärmedecke 100 hat das Merkmal, dass sich die Warmluft nach Anschalten der Warmluftquelle und nach Durchlaufen des Luftschlauchs 12 in der als Zwischenschicht ausgebildeten Zwischenlage 16 verteilt, die in der insgesamt dreilagig ausgebildeten Wärmedecke 100 zwischen der als Innenschicht ausgebildeten, dem Körper zugewandten Innenlage 14 und der als Außenschicht ausgebildeten, vom Körper abgewandten Außenlage 15 eingebracht ist (die Zwischenlage oder Zwischenschicht 16 wird also in erfindungswesentlicher Weise durch die körperzugewandte Innenlage oder Innenschicht 14 sowie durch die körperabgewandte Außenlage oder Außenschicht 15 begrenzt). Die Innenschicht bzw. Innenlage 14 ist luft- und feuchtigkeitsdurchlässig sowie hydrophil. Um dies zu gewährleisten kann die Innenschicht bzw. Innenlage 14 aus Mikrofasertuch bestehen und geschlossen sein, so dass eine feine Verteilung der Luft gewährleistet ist. Ein starker Luftstrom ist somit ausgeschlossen.

Durch das Vorsehen der Zwischenlage 16 kann die von der Warmluftquelle zur Verfügung gestellte Warmluft in jedem Falle kontinuierlich unter dem zu wärmenden Körper hindurch geführt werden, so dass die auf der Wärmedecke 100 auflagernden Körperteile unter jeglichen Bedingungen zuverlässig gewärmt werden.

Hierzu ist der innenliegende Raum oder "Innenraum" der Wärmedecke 100 mit einem Zwischenlagenmaterial aus Kunststoffgewebe, nämlich aus Polyestergewirke, gefüllt; dieses Material der Zwischenlage 16 ist in erfindungswesentlicher Weise einerseits so fest, stabil und starr, dass der Durchlaß von Warmluft sichergestellt ist, andererseits weist eben dieses Material der Zwischenlage 16 in erfindungswesentlicher Weise einen Elastizitätsgrad auf, der ein druckentlastendes Lagern von narkotisierten Patientinnen und Patienten, insbesondere von Babys und von Kleinkindern, gewährleistet.

Des Weiteren werden sowohl die Innenlage oder Innenschicht 14 als auch die Außenlage oder Außenschicht 15 durch das Anordnen der Zwischenlage oder Zwischenschicht 16 nicht so sehr zusammengedrückt, dass ein Durchlassen, ein Durchleiten, ein Durchströmen und/oder ein Transportieren der Warmluft nicht mehr möglich ist (die seitlichen Körperpartien werden ebenfalls durch die aus der Innenlage 14 ausströmende Warmluft gewärmt).

Die Außenlage 15 der Wärmedecke 100 ist hierbei luftdicht sowie feuchtigkeitsdicht und luftundurchlässig ausgebildet, wozu die Außenlage 15 aus einer Beschichtung, vorzugsweise aus Polyurethan, besteht, die direkt auf die Zwischenschicht 16 aufgetragen ist. Prinzipiell kann jedoch jedes Gewebe, das luft- und feuchtigkeitsundurchlässig ist, auf die Zwischenschicht aufgetragen werden. Hierzu gehören beispielsweise auch Laminate oder Membranbeschichtungen. Im Vergleich zu den aus dem Stand der Technik bekannten Wärmedecken zeichnet sich die erfindungsgemäße Wärmedecke 100 also dadurch aus, dass die Außenlage 15 in Form einer Beschichtung, welche direkt auf eine Zwischenschicht aufgetragen wird, die Außenlage aus einem Textilmaterial einer Wärmedecke bzw. Wärmeunterlage aus dem Stand der Technik, ersetzt. Die Innenlage 14 der Wärmedecke 100 ist insofern luftdurchlässig ausgebildet, als diese Innenlage 14 aus Mikrofasermaterial besteht und in erfindungswesentlicher Weise feinste ausgebildete Öffnungen zum Ausströmen der zugeführten Warmluft aufweist.

Die vorgenannten technischen Maßnahmen führen dazu, dass die Warmluft im Wesentlichen ausschließlich an der Innenlage 14 austreten kann, die beim Einsatz der Wärmedecke 100 dem Körper einer Patientin oder eines Patienten zugewandt ist. Auf diese Weise kann die als Wärmeunterlage fungierende Wärmedecke 100 gleichzeitig für eine Isolierung gegen Wärmeverlust und zusätzlich für eine aktive Wärmezufuhr sorgen.

Die luftdurchlässige Innenlage 14 der Wärmedecke 100 ist in für Operationsbereiche üblichem Grünton gestaltet. Im Gegensatz hierzu ist die luftdichte und luftundurchlässige Außenlage 15 in Form einer vorzugsweise aus Polyurethan bestehenden Beschichtung in heller Farbe, insbesondere in Weiß gehalten, damit diese Außenlage 15 weniger wärmeabsorbierend sowie stärker wärmereflektierend ist und sich demzufolge weniger stark erwärmt.

Eine kühlere Außenfläche 15 hat zudem den Vorteil, dass diese Außenfläche 15 beim Fixieren der Wärmedecke 100 weniger leicht durch die Rückstände von Klebestreifen, von Tapes oder von dergleichen verschmutzt werden kann, weil die Kleberückstände bei niedrigen Temperaturen weniger stark löslich sind und mithin im wesentlichen am Klebestreifen, am Tape oder an dergleichen und eben nicht an der Außenlage 14 der Wärmedecke 100 haften bleiben.

Die wiederverwendbare Wärmedecke 100 ist also für die konvektive Körpererwärmung bestimmt und kann zum Beispiel bei Operationen unter dem Körper der Patientin oder des Patienten ausgebreitet werden.

Die Wärmedecke 100 ist im ausgebreiteten Zustand dargestellt. Diese Wärmedecke 100 weist verschiedene, besonders geformte Bereiche auf, wobei den "Mittelpunkt" der Wärmedecke 100 ein Oberkörperbereich 17 bildet, der - wie auch bei bekannten Wärmedecken - auf der Brust und/oder unter dem Rücken einer Patientin oder eines Patienten anzuordnen ist.

Der Oberkörperbereich 17 ist durch einen Unterkörperbereich 20 fortgesetzt; dieser Unterkörperbereich 20 kann den Bauch und auch die Hüftpartie und auch die Gesäßpartie und auch die Beine einer Patientin oder eines Patienten abdecken.

Hervorzuheben ist auch ein kapuzenartig ausgebildeter zweiteiliger Kopfbereich 21, 22,
-- der partiell mit dem Kopf und mit dem Hals einer auf der Wärmedecke 100' zu lagernden und von der Wärmedecke 100' zu bedeckenden Person zum Erwärmen mittels austretender Warmluft in Kontakt bringbar ist und
-- der als Umfassungsprofil partiell für den Kopf und für den Hals ausgebildet ist.

Des Weiteren weist die Wärmedecke 100 seitenteilartig ausgebildete Armbereiche 18 bzw. 19 auf, die mit dem rechten Arm bzw. mit dem linken Arm einer auf der Wärmedecke 100 zu lagernden und von der Wärmedecke 100 zu bedeckenden Person zum Erwärmen mittels austretender Warmluft in Kontakt bringbar sind, indem der rechte Arm vom in der Aufsichtdarstellung der Figur 1 linken Armbereich 18 sowie der linke Arm vom in der Aufsichtdarstellung der Figur 1 rechten Armbereich 19 umschlossen wird.

Da die Armbereiche 18, 19 im Wesentlichen in Form von dreieckigen Lappen ausgestaltet sind und demzufolge auch über eine in Bezug auf die Aufsichtdarstellung gemäß Figur 1 auf dem Rücken liegende Person geklappt bzw. gelegt werden können, dienen die Armbereiche 18, 19 auch als eine Art "Zudecke", mit der die Wärmedecke 100 ausgestattet ist.

Nicht zuletzt für diesen Fall der Verwendung als "Zudecke" weisen die vorstehend beschriebenen Armbereiche 18, 19 in ihren jeweiligen Endbereichen (= Bereich des in etwa rechten Winkels der im wesentlichen dreieckförmigen Lappen) Verbindungsmittel in Form von Klettverbindungen 30, 31 auf, wobei dem in der Aufsichtdarstellung der Figur 1 linken Armbereich 18 zwei beabstandet zueinander angeordnete flauschförmige Klettbandabschnitte 30 und dem in der Aufsichtdarstellung der Figur 1 rechten Armbereich 19 ein hierzu korrespondierender hakenförmiger Klettbandabschnitt 31 zugeordnet sind, durch die die freien Enden der Armbereiche 18, 19 zum Bilden einer "Zudecke" aneinander fixiert werden können.

Durch die endseitig angeordneten Verbindungsmittel 30, 31 können die beiden Armbereiche 18, 19 nach Verlegen um den Korpus (= Brustbereich und/oder Rückenbereich) der Patientin oder des Patienten herum miteinander verbunden werden, um eine Oberkörperumhüllung fester Länge zu bilden, die ihre Konfiguration und Position stabil beibehält.

In diesem Zusammenhang sind die klettartigen Verbindungsmittel 30, 31 an den Enden der beiden Armbereiche 18, 19 so angeordnet bzw. eingerichtet, dass die Verbindungsmittel 30, 31 ein Verbinden der Endbereiche der Armbereiche 18, 19 unter verschiedenen Winkeln erlauben; dies ermöglicht es, die beiden Armbereiche 18, 19 in verschiedenen Konfigurationen und auf verschiedene Weisen um den Oberkörper oder Torso der Patientin oder des Patienten herum anzulegen und dennoch für eine sichere lösbare Verbindung der Endbereiche zu sorgen.

Schließlich weist die Wärmedecke 100 unterhalb des Oberkörperbereichs 17, das heißt im Unterkörperbereich 20 mindestens zwei nebeneinander angeordnete, taschenartig ausgebildete Beinbereiche 23 bzw. 24 auf, die dem rechten Bein bzw. dem linken Bein einer in Bezug auf die Aufsichtdarstellung gemäß Figur 1 auf dem Rücken liegenden Person zugeordnet sind.

Diese Beinbereiche 23 bzw. 24 sind in erfindungswesentlicher Weise in Form von Beinsäcken ausgebildet und/oder können in erfindungswesentlicher Weise mittels geeigneter Verschlussmittel, wie etwa mittels Klettverschlüssen 32 bzw. 33, aneinander lösbar fixiert sowie verschlossen werden, so dass die zugeführte Warmluft im Bereich der Beine verbleibt und nicht nach außen verloren geht.

Die Beinbereiche 23 bzw. 24 sind zumindest partiell mit dem jeweiligen Bein, insbesondere zumindest partiell mit dem jeweiligen Fuß, einer auf der Wärmedecke 100 zu lagernden und von der Wärmeunterlage 100 zu bedeckenden Person zum Erwärmen mittels austretender Warmluft in Kontakt bringbar, wobei das jeweilige Bein, insbesondere der jeweilige Fuß, vom jeweiligen Beinbereich 23 bzw. 24 umschlossen wird.

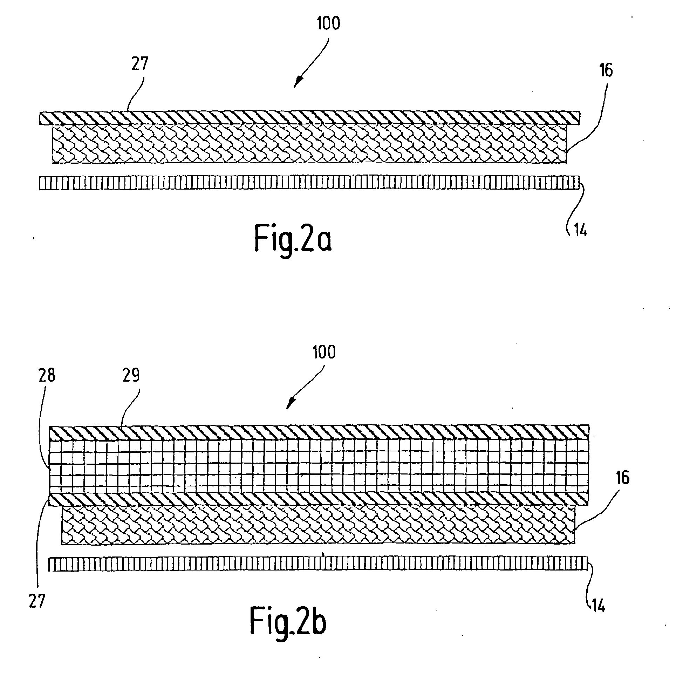

In Fig. 2a) ist ein Schnitt durch eine Wärmedecke 100 gemäß der Erfindung dargestellt, bei der Polyurethan auf die Zwischenschicht 16 aufgetragen ist. Bei der Zwischenschicht 16 kann es sich um ein Gewebe und/oder ein Gewirke handeln, welches mit der Innenschicht 14 vernäht sein kann. Die Zwischenschicht 16, die in Gestalt eines Gewirkes vorliegen kann, wird somit durch die körperzugewandte Innenschicht 14 sowie durch die Polyurethanschicht 27 begrenzt. Durch das Auftragen des Polyurethans auf die Zwischenschicht 16 und die dadurch entstehende Polyurethanschicht 27 ist eine Beschichtung gegeben, die die separate Außenschicht 15 der Wärmedecke 10 ersetzt. Die Aufbringung der Polyurethanschicht 27 kann in üblicher Weise im Streich- oder Umkehrverfahren erfolgen. Auf diese Weise geht das Polyurethan eine direkte Verbindung mit der als Gewirke ausgebildeten Zwischenschicht 16 ein. Es bleibt festzuhalten, dass die Polyurethanschicht 27 die gesamte in Figur 1 dargestellte Außenschicht 15 der Wärmedecke 10 ersetzen kann. Somit kann bei der herkömmlichen Wärmedecke 10 fortan auf die aus Polyurethan und Polyester zusammengesetzte Außenschicht 15 verzichtet werden. Die Polyurethanschicht 27 kann eine Schichtdicke von 1 bis 5 mm aufweisen. Polyurethanschichten dieser Schichtdicke zeichnen sich durch eine hohe Luftundurchlässigkeit aus.

Aus Fig. 2b) geht hervor, dass zunächst die Zwischenschicht 16, die auch hier als Gewirke vorliegen kann, mit einem Auftrag einer ersten Polyurethanschicht 27 komplett beschichtet sein kann, um hierdurch zunächst eine luftundurchlässige Schicht zu schaffen. Die Beschichtung kann auch hier im Streich- oder Umkehrverfahren erfolgen. Die Polyurethanschicht 27 kann dabei mit einer weiteren Schicht verbunden sein, die sich aus Polyurethanschaum 28 zusammensetzt. Auf den Polyurethanschaum 28 ist wiederum eine Polyurethanschicht 27 aufgetragen. Auf diese Weise entsteht gewissermaßen eine Sandwichstruktur, die mit dem Polyurethanschaum 28 einen Kern und mit den Polyurethanschichten 27 Deckschichten aufweist. Die Polyurethanschicht 27, die direkt auf die als Gewirke ausgebildete Zwischenschicht 16 aufgetragen ist, stellt zunächst die luftundurchlässige Schicht für die Wärmedecke 100 dar. Mit dem Auftragen des Polyurethanschaums 28 ist es dann in Kurzzeit möglich, größere Schichten aufzubauen, die durch Streich- bzw. Umkehrverfahren nicht zu bewerkstelligen sind. Der Auftrag einer weiteren Polyurethanschicht 27 dient der weiteren Dichtheit des Sandwichaufbaus und somit auch einer weiteren erhöhten Luftundurchlässigkeit.

### Bezugszeichenliste

- 100: Wärmedecke
- 10: Wärmedecke aus dem Stand der Technik
- 11: Anschluss
- 12: Luftschlauch
- 13: Warmluftquelle
- 14: Innenschicht
- 15: Außenschicht
- 16: Zwischenschicht
- 17: Brustbereich
- 18: Armbereich
- 19: Armbereich
- 20: Unterkörperbereich
- 21: Kopfbereich
- 22: Kopfbereich
- 23: Beinbereich
- 24: Beinbereich
- 25: Klettverschluss
- 27: Polyurethanschicht
- 28: Polyurethanschaum
- 29: Polyurethanschicht
- 30: Verbindungsmittel
- 31: Verbindungsmittel
- 32: Klettverschluss
- 33: Klettverschluss

## Patentansprüche

1. Wärmedecke (100), insbesondere eine Wärmeunterlage, aufweisend
- mindestens eine dem mittels der Wärmedecke (100) zu wärmenden Körper zugewandte, für die Warmluft durchlässig ausgebildete Innenschicht (14); und
- mindestens eine Zwischenlage oder Zwischenschicht (16) zum Durchlassen, zum Durchleiten, zum Durchströmen und/oder zum Transportieren der Warmluft,
**dadurch gekennzeichnet, dass** eine Beschichtung auf die Zwischenschicht (16) aufgetragen ist.

2. Wärmedecke nach Anspruch 1, **dadurch gekennzeichnet, dass** die Beschichtung aus Polyurethan ist.

3. Wärmedecke nach Anspruch 1 oder 2, **dadurch gekennzeichnet, dass** das Material der Zwischenschicht (16) zumindest partiell durch mindestens ein Gewebe und/oder durch mindestens ein Gewirke, insbesondere zumindest partiell durch mindestens ein Kunststoffgewebe und/oder durch mindestens ein Kunststoffgewirke, im Speziellen zumindest partiell durch mindestens ein Polyestergewebe und/oder durch mindestens ein Polyestergewirke, gebildet ist.

4. Wärmedecke nach einem der Ansprüche 1 bis 3, **dadurch gekennzeichnet, dass** eine Polyurethanschicht (27) aufgetragen ist.

5. Wärmedecke nach Anspruch 2 oder 3, **dadurch gekennzeichnet, dass** ein Polyurethanschaum (28) aufgetragen ist.

6. Wärmedecke nach Anspruch 4, **dadurch gekennzeichnet, dass** auf die Polyurethanschicht (27) Polyurethanschaum (28) aufgetragen ist und auf den Polyurethanschaum (28) eine zweite Schicht Polyurethan (29) aufgetragen ist.

7. Wärmedecke nach einem der Ansprüche 2 bis 6, **dadurch gekennzeichnet, dass** die Polyurethanschichten (27, 29) eine Schichtdicke von 1 bis 5 mm aufweisen und der Polyurethanschaum (28) in einer Dicke von 1 bis 3 cm aufgebracht ist.

8. Wärmedecke nach einem der Ansprüche 1 bis 7, **dadurch gekennzeichnet, dass** das Material der Zwischenschicht (16)
- sowohl so elastisch ausgebildet ist, dass der zu wärmende Körper druckentlastend lagerbar ist,
- als auch so starr ausgebildet ist, dass die Wärmedecke (100) durch den zu wärmenden Körper nur in einem das Durchlassen, das Durchleiten, das Durchströmen und/oder das Transportieren der Warmluft durch die Zwischenschicht (16) erlaubende Maße zusammendrückbar ist.

9. Wärmedecke nach einem der vorgehenden Ansprüche, **dadurch gekennzeichnet, dass** die Wärmedecke mindestens einen Kopfbereich (21, 22) aufweist,
- der zumindest partiell mit dem Kopf und/oder zumindest partiell mit dem Hals einer auf der Wärmedecke (100) zu lagernden und/oder von der Wärmedecke (100) zu bedeckenden Personen zum Erwärmen austretender Warmluft in Kontakt bringbar ist und/oder
- der zumindest eine zum Unterdrücken des Austretens von Warmluft in bestimmten Bereichen vorgesehen Positionen veränderliche Abdeckung aufweist und/oder
- der als mindestens ein Umfassungsprofil zumindest partiell für den Kopf und/oder zumindest partiell für den Halt einer auf der Wärmedecke (100) zu lagernden und/oder von der Wärmedecke (100) zu bedeckenden Person ausgebildet ist.

10. Wärmedecke nach einem der vorgehenden Ansprüche, **dadurch gekennzeichnet, dass** die Wärmedecke (100) mindestens einen Armbereich (18, 19) aufweist, der zumindest partiell mit dem Arm oder zumindest partiell mit beiden Armen einer auf der Wärmedecke (100) zu lagernden und/oder von der Wärmedecke (100) zu bedeckenden Person zum Erwärmen mittels austretender Warmluft in Kontakt bringbar ist, insbesondere partiell den Arm oder zumindest partiell beide Arme umschließend anordbar ist.

11. Wärmedecke nach einem der vorgehenden Ansprüche, **dadurch gekennzeichnet, dass** die Wärmedecke (100) mindestens einen Oberkörperbereich aufweist, der zumindest partiell mit der Brust und/oder zumindest partiell mit dem Rücken einer auf der Wärmedecke (100) zu lagernden und/oder von der Wärmedecke (100) zu bedeckenden Person zum Erwärmen mittels austretender Warmluft in Kontakt bringbar ist, insbesondere zumindest die Brust und/oder zumindest partiell den Rücken umschließend anordbar ist.

12. Wärmedecke gemäß einem der vorgehenden Ansprüche, **dadurch gekennzeichnet, dass** die Wärmedecke mindestens einen Unterkörperbereich (20) aufweist, der zumindest partiell mit dem Unterkörper und/oder zumindest partiell mit mindestens einem Bein, insbesondere zumindest partiell zumindest partiell mit einem Fuß, einer auf der Wärmedecke (100) zu lagernden und/oder von der Wärmedecke (100) zu bedeckenden Person zum Erwärmen mittels austretenden Warmluft in Kontakt bringbar ist, insbesondere zumindest partiell den Unterkörper und/oder zumindest partiell das Bein bzw. partiell den Fuß umschließend anordbar ist.
